(19)

Europäisches
Patentamt
European
Patent Office
Office européen
des brevets

(11) EP 2 335 577 A1

(12) EUROPEAN PATENT APPLICATION

(43) Date of publication:
22.06.2011 Bulletin 2011/25

(51) Int Cl.:
A61B 5/107 (2006.01) A61B 5/053 (2006.01)

(21) Application number: 10190529.7

(22) Date of filing: 09.11.2010

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME

(30) Priority: 21.12.2009 JP 2009288798

(71) Applicant: Tanita Corporation
Tokyo 174-8630 (JP)

(72) Inventor: Kasahara, Yasuhiro
Itabashi-ku Tokyo 174-8630 (JP)

(74) Representative: Haley, Stephen
Gill Jennings & Every LLP
Broadgate House
7 Eldon Street
GB-London EC2M 7LH (GB)

(54) Body index apparatus

(57) A body index apparatus (100) has a measurer for measuring, at plural measurement points, the distance indicating the width of abdomen (11) of a human subject (10) who lies on a reference plane (S) in the supine position. The distances from these measurement points to the reference plane (S) are different from one another. The measurement points are included in a plane (G) intersecting the reference plane (S) and are positioned so that the maximum width of the abdomen (11) in the plane (G) is between a measurement point that is the closest to the reference plane (S) and a measurement point that is the most distant from the reference plane (S). The body index apparatus (100) estimates an obesity index by calculation using the value of the ratio of the first distance to the second distance that is a distance at a predetermined measurement point, the first distance indicating the maximum width of plural distances measured at the plural measurement points.

FIG. 1

**Description**

**BACKGROUND OF THE INVENTION**

1. Field of the Invention

The present invention relates to a body index apparatus for estimating an index (obesity index) relating to obesity.

2. Description of Related Art

Obesity indices include visceral fat area and abdominal subcutaneous fat area. There is disclosed in Japanese Patent Application Laid-Open Publication No. 2009-22482 (hereinafter referred to as JP 2009-22482) a body composition measuring apparatus for estimating viscera fat area and abdominal subcutaneous fat area. This body composition measuring apparatus, with focus on a transverse plane, or a cross-section, of the abdomen of a human subject in the standing position, measures the abdominal width of the focused plane by measuring the width at plural positions that are in the anteroposterior direction of the abdomen, and performs calculations using, from among the measured abdominal widths, the maximum width (a width corresponding to the maximum width of a human subject who is in the standing position) to estimate an index relating to the body composition such as visceral fat area and abdominal subcutaneous fat area. The maximum width in the standing position is the maximum width among the abdominal widths in the focusing plane.

Another obesity index is an obesity type showing a type of obesity. Obesity types include, for example, visceral fat obesity in which visceral fat is the main factor, subcutaneous fat obesity in which subcutaneous fat is the main factor, and an intermediate type which is characterized somewhere in between. In estimating obesity type, the value of the ratio of abdominal subcutaneous fat to fat in the abdominal cavity is important. The fat in the abdominal cavity includes visceral fat and abdominal subcutaneous fat. In the estimation using the body composition measuring apparatus according to JP 2009-22482, however, an index is estimated by calculation without using the feature amount indicating this value. For example, even if the widths corresponding to the maximum width in the standing position are the same for plural subjects, the amount of visceral fat or the amount of abdominal subcutaneous fat may differ depending on whom the subject is. Therefore, the abdominal width is not a feature amount that indicates the value of the above ratio. Thus, the estimation of obesity type was difficult.

Furthermore, in the calculation in which the feature amount indicating the value of the ratio is not used, accuracy in the estimation of visceral fat area and abdominal subcutaneous fat area is suppressed to a low degree. Furthermore, no technique is known for easily measuring the feature amount indicating the above ratio.

**Summary of the Invention**

In view of the problems described above, the present invention has as an object to provide a body index apparatus capable of estimating an obesity index such as an obesity type by easily measuring the feature amount indicating the value of a ratio of abdominal subcutaneous fat to fat in the abdominal cavity.

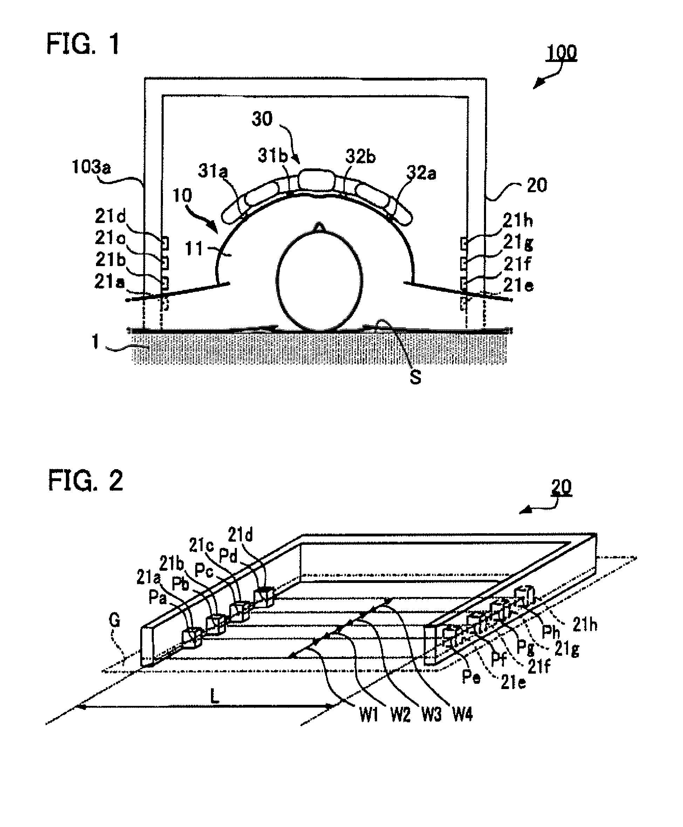

In order to solve the above-described problems, the present Invention provides a body index apparatus having a measurer (measurement means) for measuring, at plural measuring points, a distance indicating an abdominal width of a human subject lying on a reference plane in the supine position, the plural measurement points having different distances to the reference plane and being included in a plane (plane G in Fig. 2) intersecting the reference plane; and an estimator for estimating an index relating to obesity (obesity index) by calculation using a variable (variable A) relating to a difference between a first distance and a second distance, the first distance corresponding to the maximum width of plural distances at the plural measurement points measured by the measurer and the second distance corresponding to a distance of the width of a predetermined measurement point of the plural measurement points, and the plural measurement points are determined in such a way that the maximum value of the abdominal widths in the plane intersecting the reference plane is located between a measurement point closest to the reference plane and a measurement point that is the most distant from the reference plane. Plane G is a plane crossing the abdomen of a human subject who is in the supine position when a distance is measured. The subcutaneous fat in the abdomen of a human subject who is in the supine position is weighed down in the posterior direction of the abdomen by gravity. The degree of this sagging becomes more significant as the ratio (proportion B) of abdominal subcutaneous fat to fat in the abdominal cavity is greater. In other words, there is a correlation between the abdominal shape of a human subject who is in the supine position and proportion B. Therefore, using the feature amount indicating the abdominal shape in plane G of a human subj ect in the supine position enables estimation of an obesity index such as an obesity type.

This body index apparatus has a configuration for estimating an obesity index by calculation using a variable A relating to the difference between the first distance and the second distance. The first distance is a distance indicating a width corresponding to the maximum width in the supine position (maximum supine width) from among the abdominal widths in plane G of a human subject who is in the supine position, and the second distance is a distance indicating a width at a predetermined measurement point included in plane G. Therefore, variable A is the tendency of the abdominal width distribution in plane G of a human subject in the supine position, i.e., the feature amount indicating the abdominal shape in plane G of a human subject who is in the supine position. Furthermore, as described above, there is a correlation between the abdominal shape of a human subject who is in the supine position and proportion B. In other words, variable A is the feature amount indicating the value of proportion B. Therefore, according to this body index apparatus, an obesity index such as an obesity type can be estimated by measuring the feature amount indicating the value of proportion B (the ratio of abdominal subcutaneous fat to fat in the abdominal cavity).

Moreover, in this body index apparatus, a human subject simply needs to be in the supine position. Therefore, the feature amount indicating the value of proportion B can be measured even if a human subject is bedridden. Furthermore, because the measurer is a means for measuring a distance indicating the abdominal width at plural measurement points, the configuration can be simplified. Therefore, according to this body index apparatus, the feature amount indicating the value of proportion B (the ratio of abdominal subcutaneous fat to fat in the abdominal cavity) can be easily measured.

As described above, the subcutaneous fat in the abdomen of a human subject who is in the supine position is weighed down in the posterior direction of the abdomen. Therefore, in view of improving the degree of accuracy in estimation, it is preferable to make a plane perpendicular to the vertical direction a reference plane and to measure the distance by positioning the measurer so that plane G is perpendicular to this reference plane. In this case, it is preferable that the measurer be positioned so that plane G is also perpendicular to the inferior-superior direction of a human subject during the distance measurement.

Furthermore, the measurer has, for example, plural pairs of distance measuring sensors configured of two distance measuring sensors facing each other, sandwiching the abdomen in the abdominal width direction (transversal direction), and has a summing unit (summing means) for calculating a total distance by summing, for each pair of distance measuring sensors, distances measured by two distance measuring sensors of each pair. In this summing unit, plural pairs of distance measuring sensors are positioned respectively at plural measurement points, and each distance measuring sensor measures a distance to the abdomen at the measurement point at which the sensor is positioned. In this case, each of plural total distances may be used as each of the "plural distances at the plural measurement points", the total distance being obtained by summing, for each pair of distance measuring sensors, distances (two values of distances) measured by the distance measuring sensors in the pair. Alternatively, the difference between the total distance for each pair of distance measuring sensors and the distance between two distance measuring sensors configuring the same pair of distance measuring sensors may be used as each of the "plural distances at the plural measurement points".

Additionally, the "predetermined measurement point" may be either one or plural. In a case in which the predetermined measurement point is plural, calculation for estimating an index is calculation corresponding to each of the plural predetermined measurement points and to an index to be estimated. Furthermore, exemplary calculations for estimating an obesity type include a calculation using at least one of a conditional equation or a regression equation corresponding to a predetermined measurement point. The threshold in a conditional equation or the coefficients in a regression equation is determined so that the degree of accuracy in estimation will be the highest when the first distance indicates the maximum supine width.

In the above body index apparatus, the estimator may execute calculation using the first distance and the variable (variable A). The first distance is a distance indicating a width corresponding to the maximum supine width, and there is a strong correlation between the maximum supine width and an obesity index such as abdominal subcutaneous fat area, visceral fat area, and abdominal circumference. Therefore, according to this body index apparatus, an obesity index such as abdominal subcutaneous fat area, visceral fat area, and abdominal circumference can be estimated with a high degree of accuracy. The abdominal circumference is a circumferential length of a cross-section of the abdomen.

Description is now given of a reason that the abdominal circumference can be estimated with a high degree of accuracy. In estimating the abdominal circumference, no distinction is normally needed between the visceral fat and the abdominal subcutaneous fat. However, the subcutaneous fat of the abdomen in a human subject is weighed down when the human subject is in a supine position. Therefore, the width at a position closer to the back of a human subject could be considerably longer than the maximum width in the standing position. In this case, the first distance could indicate a width that is considerably longer than the maximum width in the standing position. In such a case, if only the first distance is used in the calculation for estimating the abdominal circumference, there could be a significant discrepancy between the estimated value and the actually measured value. In contrast, in the body index apparatus of the present invention, not only the first distance but also variable A being the feature amount indicating the abdominal shape is used in the calculation for estimating the abdominal circumference. Therefore, the above discrepancy can be suppressed. This is a reason that the abdominal circumference can be estimated with a high degree of accuracy.

The above body index apparatus may be additionally provided with a bioelectrical impedance measurer (bioelectrical impedance measurement means) for measuring bioelectrical impedance of the abdomen by bringing plural electrodes into contact with the abdomen, and the estimator may execute calculation using the bioelectrical impedance measured by the bioelectrical impedance measurer, the first distance, and the variable. There is a strong correlation between an obesity index such as abdominal subcutaneous fat area and visceral fat area, and the bioelectrical impedance of the abdomen. Therefore, according to this body index apparatus, the degree of accuracy in estimation is enhanced in estimating an obesity index such as abdominal subcutaneous fat area and visceral fat area.

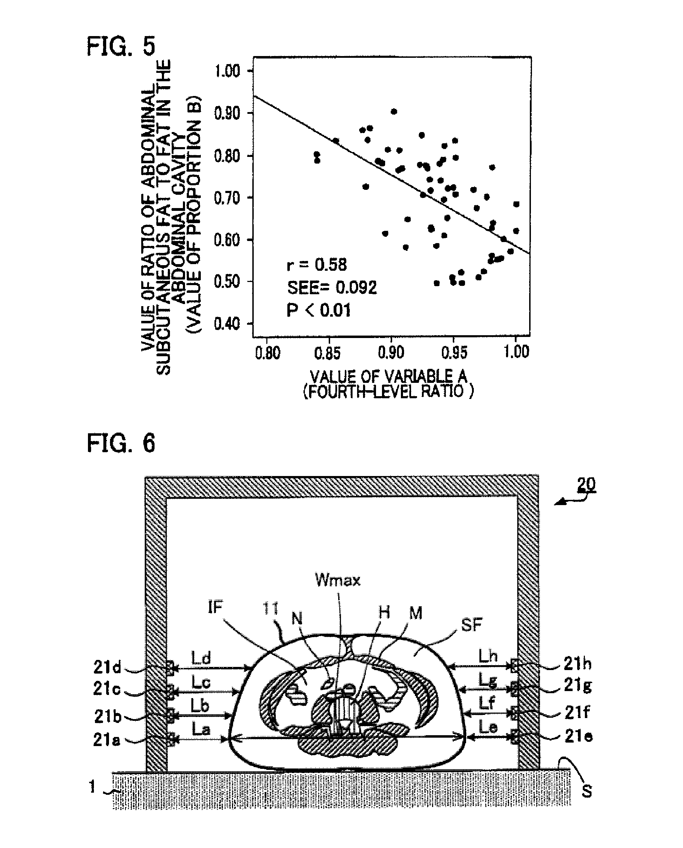

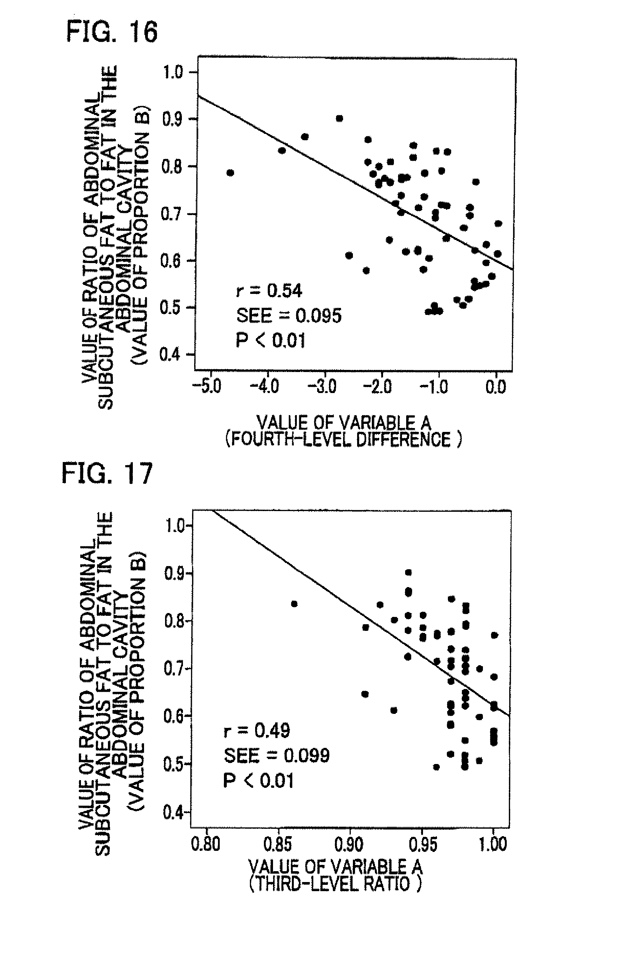

The variable (variable A) may be, for example, the ratio between the first distance and the second distance or the difference between the first distance and the second distance. As shown in Fig. 5, there is a correlation between variable A and proportion B, in which case variable A is the ratio of the second distance to the first distance. Furthermore, as shown in Fig. I6, there is a correlation between variable A and proportion B, in which case variable A is the difference between the first distance and the second distance. Thus, in either case, an obesity index can be estimated. Nevertheless, as is clear from the comparison between Figs. 5 and 16, the ratio is preferred to the difference in view of improving the accuracy in the estimation of an obesity index.

As described above, in the present invention, the abdominal shape of a human subject who is in the supine position is taken into consideration in estimating an obesity index. If the shape indicated by variable A is very different from the actual abdominal shape, the accuracy in estimating an obesity index is degraded. In view of preventing variable A indicating the shape from being very different from the actual abdominal shape, it is suitable to use, from among the plural measurement points, a measurement point that is the closest to the reference plane or one that is the most distant from the reference plane as the predetermined measurement point. The rationales are as follows:

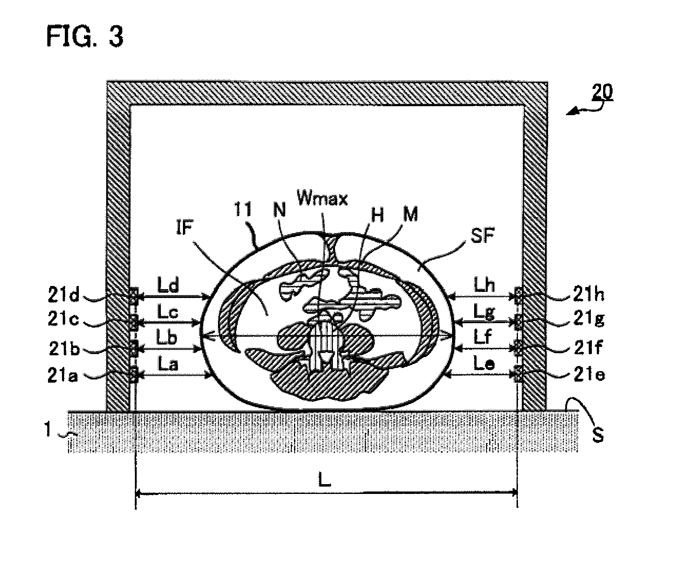

For example, as shown in Fig. 3, arranged sequentially from a reference plane (reference plane S) are a first measurement point (distance measuring sensors 21 a and 21e), a second measurement point (distance measuring sensors 21b and 21 f), a third measurement point (distance measuring sensors 21c and 21 g), and a fourth measurement point (distance measuring sensors 21d and 21h). It is additionally assumed that a width indicated by the second distance is longer than a width indicated by the first distance.

In this case, in a case in which the predetermined measurement point is the second measurement point, the position of the maximum supine width (Wmax) could be either the anterior side (the side of the third measurement point) of the predetermined measurement point or the posterior side (the side of the first measurement point) of the predetermined position. In a case in which the predetermined measurement point is the third measurement point, the position of the maximum supine width (Wmax) could be either the anterior side (the side of the fourth measurement point) of the predetermined measurement point or the posterior side (the side of the second measurement point) of the predetermined position.

In contrast, in a case in which the predetermined measurement point is the first measurement point that is the closest to the reference plane, the position of the maximum supine width (Wmax) could be the anterior side (the side of the second measurement point) of the predetermined measurement point only. Furthermore, in a case in which the predetermined measurement point is the fourth measurement point that is the most distant from the reference plane, the position of the maximum supine width (Wmax) could be the posterior side (the side of the third measurement point) of the predetermined measurement point only.

Thus, by using a measurement point, from among plural measurement points, that is the closest to the reference plane or a measurement point that is the most distant from the reference plane as the predetermined measurement point, candidates for the relative positional relationship between the predetermined measurement point and the maximum supine width can narrowed down in a case in which a width indicated by the first distance is longer than a width indicated by the second distance. This means that the abdominal shape, to be indicated by variable A, of a human subject in the supine position is more specified. Thus, by determining, as the predetermined measurement point, a measurement point, from among the plural measurement points, that is the closest to the reference plane or a measurement point that is the most distant from the reference plane, the possibility is reduced of the abdominal shape indicated by variable A being very different from the actual abdominal shape in a case in which a width indicated by the first distance is longer than a width indicated by the second distance.

Moreover, for the predetermined measurement point, a measurement point that is the most distant from the reference plane is preferred to a measurement point closest to the reference plane. The rationales are as follows.

Because the subcutaneous fat in the abdomen of a human subject in the supine position is weighed down in the posterior side of the abdomen by gravity, a case in which the first distance is longer than a width at a measurement point that is the most distant from reference plane S is more likely to occur than a case in which the first distance is longer than a width at a measurement point that is the closest to reference plane S. In other words, a case in which the width indicated by the first distance is longer than the width indicated by the second distance is more likely to occur when a measurement point that is the most distant from reference plane S is the predetermined measurement point in comparison with a case in which a measurement point that is the closest to reference plane S is the predetermined measurement point.

In a case in which the predetermined measurement point is a measurement point that is the most distant from the reference plane among the plural measurement points, the estimator may be capable of executing a first calculation in which the index is estimated when the predetermined measurement point is a measurement point that is the most distant from the reference plane and also capable of executing a second calculation in which the index is estimated when the predetermined measurement point is another measurement point, and the estimator may execute the second calculation in a case in which the distance of a measurement point that is measured by the measurer and is the most distant from the reference plane indicates zero, and executes the first calculation in a case in which the distance of a measurement point that is measured by the measurer and is the most distant from the reference plane does not indicate zero.

In a case in which the predetermined measurement point is a measurement point that is the most distant from the reference plane among the plural measurement points, there is a greater probability of the distance indicating zero (the distance indicating the width of zero) being measured at the predetermined measurement point. In a case in which a distance indicating the width of zero is the second distance, the distance between the measurement point at which the distance indicating the width of zero is measured and abdomen must be taken into consideration. Otherwise, the degree of accuracy in estimating the abdominal shape in the supine position is significantly degraded. However, in this body index apparatus, in a case in which the distance measured at a measurement point that is the most distant from reference plane S indicates the width of zero, the distance measured at another measurement point will be the second distance. Thus, according to this body index apparatus, a situation can be avoided in which the degree of accuracy in estimating the abdominal shape is significantly degraded.

In each of the above body index apparatuses, at least one measurement point of the plural measurement points may be movable in the direction intersecting the reference plane. As described above, the first distance is a distance indicating a width corresponding to the maximum supine width. Therefore, if the width indicated by the first distance is significantly shorter than the maximum supine width, the shape indicated by variable A would be very different from the actual shape. Conversely, according to this body index apparatus, movable measurement points are moved, whereby the width indicated by the first distance can be made closer to the maximum supine width. Thus, according to this body index apparatus, the accuracy in the obesity index estimation is enhanced.

When at least one of a regression equation or a conditional equation is used in a calculation, it is only necessary to prepare at least one of a regression equation or a conditional equation corresponding to the predetermined measurement point in a case in which the predetermined measurement point is fixed. On the other hand, in a case in which the predetermined measurement point is movable, plural sets of at least one of a regression equation or a conditional equation corresponding respectively to measurement points that could possibly be the predetermined measurement point should be prepared in advance, so as to use, for the calculation, a set of at least one of a regression equation or a conditional equation corresponding to a measurement point that is to be the predetermined measurement point.

In each of the above body index apparatuses, the measurer may measure the distance of the plural measurement points without touching the abdomen. In this body index apparatus, the distance required for calculation for estimating an index is measured without bringing the measurer into contact with the abdomen. In a contact-type measurement, the accuracy in measuring the distance is degraded because the abdomen is subject to deformation because the distance measuring sensor touches the surface. In contrast, in a contactless-type measurement, the accuracy in measurement is not degraded because such a deformation of the abdomen does not take place. Thus, according to this body index apparatus, the degree of accuracy in estimating an obesity index is enhanced.

BRIEF DESCRIPTION OF THE DRAWINGS

Fig. 1 is an elevational view showing an external view of a body index apparatus 100 according to a first embodiment of the present invention.

Fig. 2 is a perspective view showing an external view of a frame 20 of body index apparatus 100.

Fig. 3 is a cross-sectional view showing the positional relationship between frame 20 and abdomen 11 (intermediate type obesity).

Fig. 4 is a block diagram showing an electrical configuration of body index apparatus 100.

Fig. 5 is a scatter diagram showing the relationship between a variable A and a proportion B (fourth-level ratio).

Fig. 6 is a cross-sectional view showing the positional relationship between frame 20 and abdomen 11 (subcutaneous fat obesity).

Fig. 7 is a cross-sectional view showing the positional relationship between frame 20 and abdomen 11 (visceral fat obesity).

Fig. 8 is a scatter diagram showing the relationship between the actually measured value and the estimated value EW of the abdominal circumference by a regression equation using variable A.

Fig. 9 is a scatter diagram showing the relationship between the actually measured value and the estimated value EW of the abdominal circumference by a regression equation not using variable A.

Fig. 10 is a scatter diagram showing the relationship between the estimated value EIF of visceral fat area by a regression equation not using bioelectrical impedance and visceral fat area measured using a CT (computed tomography) scanning method.

Fig. 11 is a scatter diagram showing the relationship between the estimated value EIF of visceral fat area by a regression equation using bioelectrical impedance and visceral fat area measured using a CT scanning method

Fig. 12 is a scatter diagram showing the relationship between the estimation value ESF of abdominal subcutaneous fat area by a regression equation not using bioelectrical impedance and abdominal subcutaneous fat area measured using a CT scanning method.

Fig. 13 is a scatter diagram showing the relationship between the estimation value ESF of abdominal subcutaneous fat area by a regression equation using bioelectrical impedance and abdominal subcutaneous fat area measured using a CT scanning method.

Fig. 14 is a block diagram showing an electrical configuration of a body index apparatus 200 according to a second embodiment of the present invention.

Fig. 15 is a perspective view showing an external view of a frame 50 of body index apparatus 200.

Fig. 16 is a scatter diagram showing the relationship between variable A and proportion B (fourth-level difference).

Fig. 17 is a scatter diagram showing the relationship between variable A and proportion B (third-level ratio).

## DETAILED DESCRIPTION OF PREFERRED EMBODIMENTS

First embodiment

A first embodiment according to the present invention is a body index apparatus 100 having a measurer, or measurement means, in which all the plural measurement points are fixed, the measurer including a distance measuring sensor 21, a CPU 41, a ROM 42, and a memory 43 (described later). The measurer measures, at plural measurement points, distances for abdomen 11 of a human subject 10 lying on the upper side of a bed 1 in the supine position, the upper side corresponding to a reference plane S. Body index apparatus 100 additionally has a bioelectrical impedance measurer, or bioelectrical impedance measurement means, for measuring the bioelectrical impedance of abdomen 11, with the bioelectrical impedance measured including a bioelectrical impedance measurer 30, CPU 41, ROM 42, and memory 43 (described later), and an estimator, or estimation means, for estimating an obesity index based on a measured distance and a measured bioelectrical impedance, the estimator including CPU 41, ROM 42, and memory 43 (described later). Obesity indices include abdominal circumference, abdominal subcutaneous fat area, visceral fat area, and obesity type.

In the following, description will be given of body index apparatus 100 with reference to the drawings.

Fig. 1 is an elevational view showing an external view of body index apparatus 100, the view also including a human subject 10 who is lying on reference plane S in the supine position. As shown in the figure, body index apparatus 100 has a frame 20 and a bioelectrical impedance measurer 30. Frame 20 is a frame, one side of which is open, and has plural distance measuring sensors 21a to 21 h for measuring distance. In measuring distance, frame 20 is disposed on reference plane S around abdomen 11. Bioelectrical impedance measurer 30 has current supply electrodes 31 a and 32a and voltage detection electrodes 31 b and 32b, each of which is brought into contact with abdomen 11 while measuring bioelectrical impedance.

Fig. 2 is a perspective view showing an external view of frame 20. Fig, 3 is a cross-sectional view showing the positional relationship between frame 20 and abdomen 11 during distance measurement. As shown in Fig. 2, the inner side of frame 20 is provided with plural distance measuring sensors 21 fox measuring distance.

Each distance measuring sensor 21 is an optical distance measuring sensor, and, for example, has a light emitter for emitting a light such as an infrared light beam and has a light receiver for receiving the light reflected from a point to be measured. Each distance measuring sensor 21 outputs an electric signal corresponding to a gap distance between a reference point P within the sensor and a point to be measured. In the following description, the appended letters "a" to "h" are used to distinguish each distance measuring sensor 21 and each reference point P.

For example, distance measuring sensor 21 a outputs an electric signal corresponding to a gap distance between a preference point Pa and a point to be measured (La in Fig. 3). Similarly, distance measuring sensors 21b to 21h each output electronic signals corresponding to gap distances (Lb to Lh in Fig. 3) between reference points Pb to Ph and points to be measured, respectively. A point to be measured for each distance measuring sensor 21 is a point at which the distance measuring axis of the same distance measuring sensor 21 intersects with an object such as human subject 10.

Furthermore, as shown in Fig. 2, distance measuring sensors 21b to 21h are included in plane G Specifically, distance measuring sensors 21b to 21 h are disposed in such a way that reference points Pb to Ph and points to be measured are included in plane G. Plane 0 is a plane crossing abdomen 11 of human subject 10 in the supine position while measuring distance, and extends along the array direction of distance measuring sensors 21b to 21h (for example, the direction of the plane of paper in Fig. 3). A line with an alternating long dash and two short dashes in Fig. 2 is a line of intersection between the visible side of frame 20 and plane G.

Furthermore, each distance measuring sensor 21 is provided in such a way that the distance measuring axis thereof is in parallel with reference plane S when measuring distance. Furthermore, distance measuring sensors 21a to 21d are arranged axisymmetrically (line-symmetrically) with distance measuring sensors 21e to 21h In other words, as shown in Fig. 2, the distance measuring axes of distance measuring sensors 21a and 21e are collinear; the distance measuring axes of distance measuring sensors 21b and 21f are collinear; the distance measuring axes of distance measuring sensor 21c and 21g are collinear; and the distance measuring axes of distance measuring sensor 21d and 21h are collinear.

In the following description, distance measuring sensors 21a and 21e will be referred to as a first-level pair of distance measuring sensors that are the closest to reference plane S, distance measuring sensors 21b and 21f as a second-level pair of distance measuring sensors that are the second closest to reference plane S, distance measuring sensors 21c and 21g as a third-level pair of distance measuring sensors that are the third closest to reference plane S, distance measuring sensors 21d and 21h as a fourth-level pair of distance measuring sensors that are the most distant from reference plane S. In other words, frame 20 has plural pairs of distance measuring sensors at plural measurement points, each pair consisting of two distance measuring sensors 21 facing each other in the width direction of abdomen 11 of human subject 10 and the two distance measuring sensors of the same pair sandwiching abdomen 11.

The plural measurement points are determined so that the maximum width (hereinafter referred to as "maximum supine width (Wmax)") from among widths of abdomen 11 on plane G is located between a measurement point that is the closest to reference plane S and a measurement point that is the most distant from reference plane S. For example, the distance W1 between reference plane S and the distance measuring axis of the first level is 4 cm, and the distance $W(n + 1)$ between the distance measuring axis of the n-th level and the distance measuring axis of the $(n + 1)$th level is 3 cm, where $n$ is a natural number equal to or less than 3. Furthermore, the distance (L) between two distance measuring sensors 21 making up each distance measuring sensor pair is the same for every distance measuring sensor.

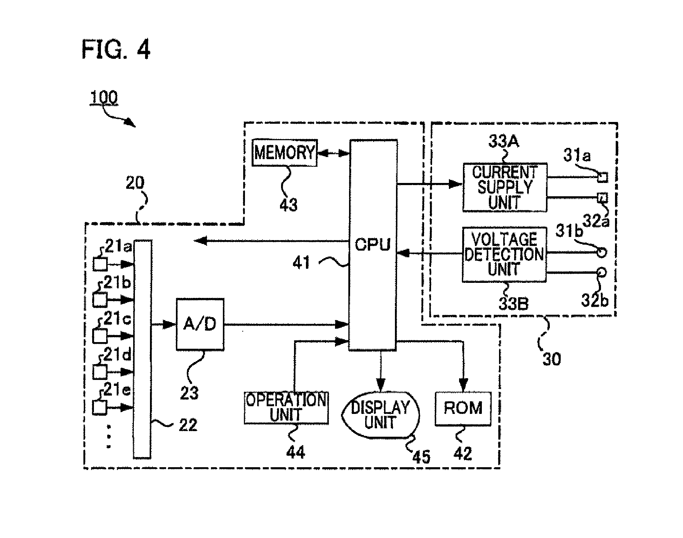

Fig. 4 is a block diagram showing an electrical configuration of body index apparatus 100. As shown in this figure, frame 20 has a switch 22 and an analog/digital converter 23 (A/D converter 23) in addition to distance measuring sensors 21a to 21h. Switch 22 sequentially selects an output signal from distance measuring sensors 21a to 21h, for supply to A/D converter 23. A/D converter 23 converts the supplied signal to a digital signal, for supply to CPU 41 (described later). Thus, when measuring distance, pieces of distance data showing distance measured by distance measuring sensors 21a to 21h are sequentially supplied from A/D converter 23 to CPU 41.

Bioelectrical impedance measurer 30 is provided with a current supply unit 33A and a voltage detection unit 33B in addition to current supply electrodes 31a and 32a and voltage detection electrodes 3 1 b and 32b. Current supply unit 33A, in measuring bioelectrical impedance, supplies to abdomen I of human subject 10 a constant current of high frequency and a constant current of low frequency via current supply electrodes 31a and 32a. The frequency of a high-frequency current is, for example, 50 kHz, and the frequency of a low-frequency current is, for example, 6.25 kHz. A period in which a high-frequency current is supplied (a high-frequency period) and a period in which a low-frequency current is supplied (a low frequency period) do not overlap with each other.

Voltage detection unit 33B, in each of the high-frequency period and the low-frequency period, measures voltage between voltage detection electrode 31b and voltage detection electrode 32b, and voltage data showing a result of this measurement (voltage) is wirelessly supplied to CPU 41 of frame 20. Both the voltage as of a time a high-frequency current is supplied and the voltage as of a time a low-frequency current is supplied are measured in order to measure bioelectrical impedance suitable for estimating visceral fat area and abdominal subcutaneous fat area, i.e., in order to measure bioelectrical impedance that is a little affected by the electrolyte tissues such as muscular tissues. The electrical resistance of non-electrolyte tissues such as fat tissues does not change much regardless of whether a low frequency or a high frequency is used, whereas the electrical resistance of electrolyte tissues considerably changes from when a low frequency is used to when a high frequency is used. Therefore, by measuring voltage of both cases, it is possible to calculate bioelectrical impedance that is a little affected by electrolyte tissues.

Furthermore, frame 20 has a CPU (central processing unit) 41, a ROM (read only memory) 42, a memory 43, an operation unit 44 operated by a person, and a display unit 45 for displaying information. Stored in ROM 42 is a computer program (hereinafter referred to as "first computer program") executed by CPU 41. Memory 43 is, for example, a volatile memory, and is used as a work area for CPU 41. Operation unit 44 supplies to CPU 41 a signal corresponding to an operation. Information displayed on display unit 45 is, for example, an estimation result of an obesity index, the estimation having been supplied from CPU 41.

CPU 41 executes she first computer program stored in ROM 42, controls each unit, and perform various types of calculations. For example, CPU 4t, when a signal instructing the start of bioelectrical impedance measurement is received from operation unit 44, controls current supply unit 33A to cause the unit to supply current to abdomen 11 for the high frequency period and for the low frequency period, receives two types of voltage data pieces from voltage detection unit 33B, calculates, based on the received two types of voltage data pieces, the bioelectrical impedance (Z) for abdomen 11, and writes bioelectrical impedance data showing the calculated bioelectrical impedance (Z) into memory 43. The calculated bioelectrical impedance (Z) is bioelectrical impedance that is a little affected by electrolyte tissues.

Furthermore for example, CPU 41, when a signal instructing the start of bioelectrical impedance measurement is received from operation unit 44, causes distance measuring sensors 21a to 21h to start measuring distances, causes switch 22 to start selection, receives distance data sequentially supplied from A/D converter 23, and calculates a total distance (La + Le, Lb + Lf, Lc + Lg, Ld + Lh) for each pair of distance measuring sensors by summing, for each pair, distances indicated by the received pieces of distance data.

CPU 41, from among the total distances, regards the shortest total distance as a first distance (L1), and writes first distance data indicating this distance into memory 43. CPU 41 regards a total distance corresponding to the fourth level pair of distance measuring sensors (a pair of distance measuring sensors at a predetermined measurement point) as a second distance (L2), calculates the value of ratio of the first distance to the second distance, and writes data showing this ratio as variable data indicating the value of a variable A into memory 43. Thus, A = L1/ L2, and variable A is the ratio of the first distance (11) to the second distance (L2). This ratio indicates the tendency in distribution of widths of abdomen 11 of human subject 10 in the supine position in plane G Therefore, variable A is the feature amount indicating the shape of abdomen 11 in plane G

An obesity index is widely used in view of maintaining good health, and is an index estimated based on the feature amount measured for a cross-section (a plane perpendicular to the inferior-superior direction) passing through the navel of the abdomen. Wherefore, in measuring bioelectrical impedance, it is preferable to locate bioelectrical impedance measurer 30 so that electrodes 31a, 32a, 31b, and 32b are arranged on a plane including a cross-section passing through the navel of abdomen 11; and in measuring a distance, it is preferable to locate frame 20 so that the plane is included in plane G. That is, it is preferable to locate bioelectrical impedance measurer 30 and frame 20 in a direction perpendicular to the inferior-superior direction. Because bioelectrical impedance measurer 30 coming into contact with abdomen 11 causes deformation of abdomen 11, it is not preferable to perform bioelectrical impedance measurement and distance measurement at the same time.

An index estimated based on the feature amount measured for a plane other than a cross-section crossing the navel of the abdomen can also be an obesity index that is useful for maintaining good health. In other words, according to the present embodiment, it is possible to estimate an obesity index useful for maintaining good health even if a cross-section passing through the navel of the abdomen is not included in plane G in measuring distance. For example, in measuring distance, a cross-section (a plane perpendicular to the interior-superior direction) not passing through the navel of the abdomen may be included in plane G. Plane G may cross the abdomen obliquely in the anteroposterior direction of the abdomen; and plane G may cross the abdomen obliquely in the abdominal width direction. Plane G may cross the abdomen obliquely both in the anteroposterior direction and in the width direction of the abdomen.

Furthermore, for example, CPU 41 estimates an obesity index by calculation using an equation when a signal is supplied from operation unit 44, the signal instructing the start of estimation for an obesity index, and causes a result of estimation to be displayed on display unit 45. An equation used for calculation for estimating an obesity index is determined in advance for each obesity index, and each equation has a term including variable A that is the feature amount of the abdomen.

Fig. 5 is a scatter diagram showing the relationship between a value of variable A and a ratio (proportion B) of abdominal subcutaneous fat area to fat area in the abdominal cavity. In this figure, the value of proportion B in this figure is calculated based on a value measured by a CT scanning (Computed Tomography scanning) method. Furthermore, in this figure, "r" is a correlation coefficient, an "SEE" is a standard error of estimate for an estimated value, and P is a risk rate. As is clear from this figure, there is a strong correlation between variable A and proportion B. Therefore, using an equation having a term including variable A in the calculation for estimating an obesity index can improve the degree of accuracy in estimating an obesity index such as an obesity type. This is why an equation having a term including variable A is used in the calculation for estimating an obesity index. It is to be noted that, because proportion B is a ratio of abdominal subcutaneous fat area to fat area in the abdominal cavity, variable A having a strong correlation with proportion B is a feature amount showing a value of ratio of abdominal subcutaneous fat to fat in the abdominal cavity.

Description is now given of different types of obesity indicated by an obesity type. In the present embodiment, a type for which the value of the above proportion B is within a predetermined range is regarded as an intermediate type; a type for which the value of proportion B is below the lower limit of the range is regarded as a visceral fat type; and a type for which the value of proportion B is above the upper limit of the range is regarded as a subcutaneous fat type. This is merely an example, and a range that is different from the above range can be the predetermined range. Alternatively or additionally, an intermediate type may be deleted. In a case in which the intermediate type is deleted, if a value of proportion B is equal to or less than a predetermined value, the obesity type will be a visceral fat type, and it will be a subcutaneous fat type if a value of proportion B is above the predetermined value.

Description will next be given of a reason for which variable A has a strong correlation with proportion B.

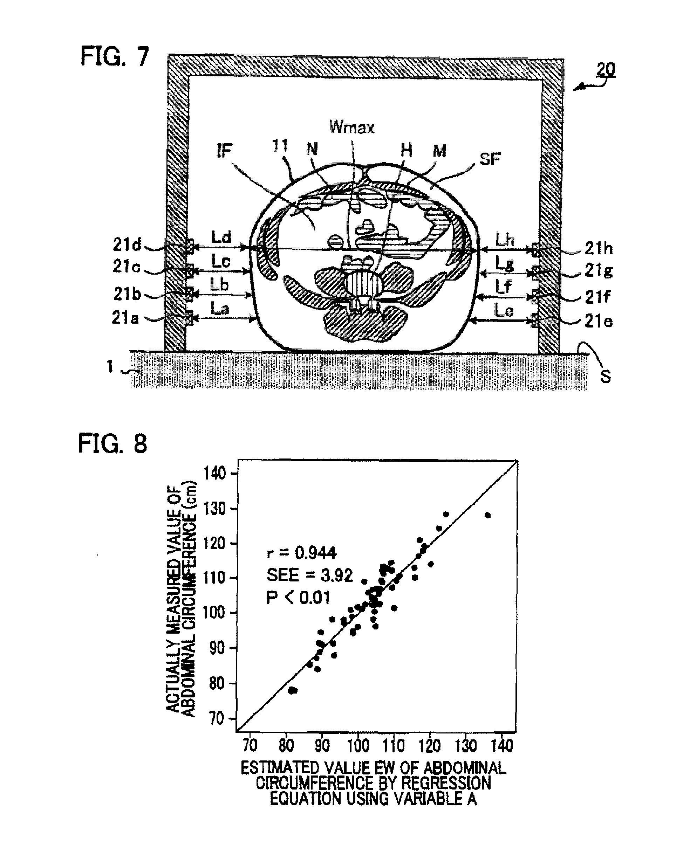

Figs. 6 and 7 are cross-sectional views each showing a positional relationship between frame 20 and abdomen 11 in the distance measurement, like in Fig. 3. However, the obesity type of human subject 10 is an intermediate type in Fig. 3, a subcutaneous fat type in Fig. 6, and a visceral fat type in Fig. 7. As shown in these figures, bone H with vertical hatching; organ N with horizontal hatching, muscle M with oblique hatching, subcutaneous fat SF (fat beneath the skin) above muscle M, and visceral fat IF beneath muscle M are visible in a cross-section of abdomen 11.

In a case in which human subject 10 is in the supine position a portion of subcutaneous fat SF not having muscle M directly therebeneath is weighed down by gravity to a posterior direction of abdomen 11. The degree of this sag is likely to be greater as the value of proportion B is greater (Fig. 6), and is likely to be smaller as the value of proportion B is smaller (Fig. 7). Therefore, the level of the maximum width Wmax in the supine position in a direction perpendicular to reference plane S (ideally, a vertical direction) tends to be lower (i.e., closer to reference plane S) as the value of proportion B is greater (Fig. 6), and tends to be higher as the value of proportion B is smaller (Fig. 7).

On the other hand, variable A is (La + Le) / (Ld + Lh) in Fig. 6 and is considerably less than 1, whereas, in Fig. 7, variable A is (Ld + Lh) / (Ld + Lh) and is equal to 1, the variable A being a ratio of the first distance to the second distance. In other words, the value (A) of variable A tends to be smaller as the level of the maximum supine width (Wmax) is lower, and to be greater as the level of Wmax is higher. That is, the value (A) of variable A tends to be smaller as the value of proportion B is greater, and tends to be greater as the value of proportion B is smaller. This is a reason for which there is a strong correlation between variable A and proportion B.

In estimating an obesity index, CPU 41 selects an equation for each index to be estimated, to perform calculation using the selected equation. Prior to the calculation, CPU 41 reads from memory 43 first distance data and variable data. Furthermore, CPU 41 reads bioelectrical impedance data if there is stored in memory 43 bioelectrical impedance data. First distance (L1), the value (A) of variable A, and bioelectrical impedance (Z) indicated by the read pieces of data, and distance (L) between the two distance measuring sensors 21 configuring each pair of distance measuring sensors are used to estimate abdominal circumference, visceral fat area, abdominal subcutaneous fat area, and obesity type.

Estimating abdominal circumference

In estimating abdominal circumference, Equation 1 is selected. Equation 1 is a regression equation using variable A.

$$EW = -68.9 + 3.13 * (L - L1) + 62.0 * A \ldots \quad \text{Equation 1}$$

Coefficients of each term of Equation 1 are determined so that the degree of accuracy in the estimation is the highest when L2 = Ld + Lh and L1 = Wmax. CPU 41 uses L1, A, L, and Equation 1, to calculate an estimated value EW of the abdominal circumference. The relationship between this estimated value EW and actually measured values of the abdominal circumference are as shown in Fig. 8. As shown in this figure, there is a strong relationship between the estimated values and the actually measured values. Thus, according to body index apparatus 100, abdominal circumference can be estimated with a high degree of accuracy.

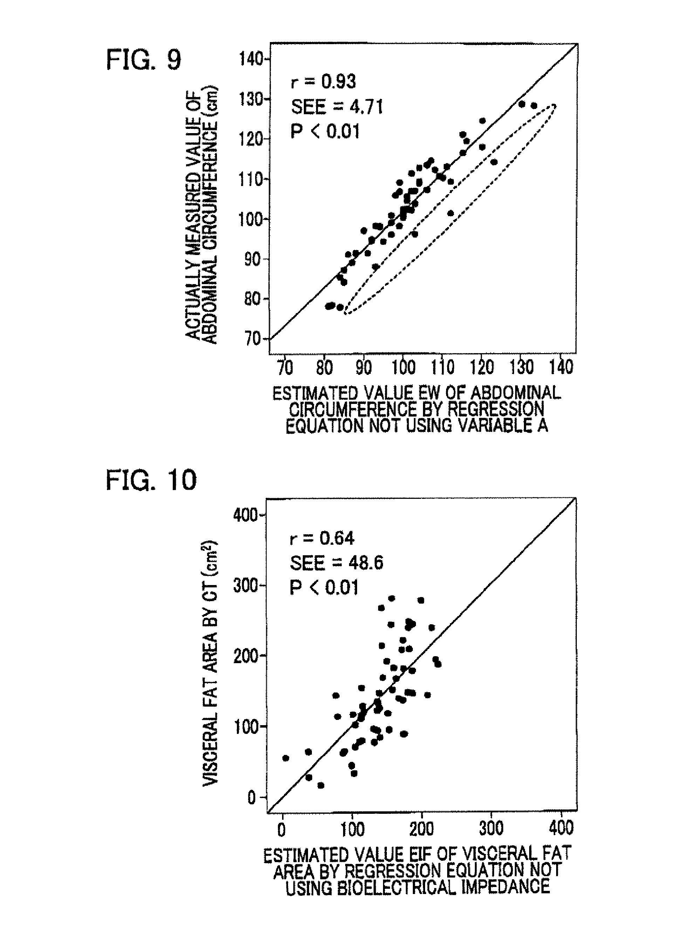

Fig. 9 is a scatter diagram showing the relationship between estimated values of the abdominal circumference according to a regression equation not using variable A and actually measured values of the abdominal circumference. In an area encircled by a dashed line of Fig. 9, there are shown samples for which estimated values are significantly discrepant from actually measured values. These samples are human subjects 10 whose proportion B is significantly high. When proportion B of human subject 10 in the supine position is significantly high, there are cases in which the width of abdomen 11 in its posterior portion due to the sagging subcutaneous fat is significantly longer than the maximum width in the standing position. In these cases, the first distance could indicate a width that is significantly longer than the maximum width in the standing position.

If variable A indicating the feature amount of the abdominal shape is not used in these cases, the estimated values of abdominal circumference and the actually measured value could considerably differ from each other. This is a reason for which there are samples for which the estimated values and actually measured values considerably differ from each other in Fig. 9. In contrast, there is no such a sample in Fig. 8. This is because the abdominal circumference can be estimated with a high degree of accuracy by calculation using an equation including variable A.

Estimating visceral fat area

In the visceral fat area estimation. Equation 2 is selected in a case in which bioelectrical impedance data is not stored in memory 43, the Equation 2 being a regression equation not using Z; and Equation 3 is selected in a case in which bioelectrical impedance data is stored in memory 43, the Equation 3 being a regression equation using Z.

$$EIF = -907.2 + 937.8 * A + 0.127 * (L - L1)^2 \ldots \text{Equation 2}$$

$$EIF = -787.4 + 878.6 * A + 0.001215 * Z * (L - L1)^2 \ldots \text{Equation 3}$$

The coefficients in each term of Equation 2 and Equation 3 are determined so that the degree of accuracy in estimation is the highest when L2 = Ld + Lh and L1 = Wmax.

In a case in which bioelectrical impedance data is not stored in memory 43, CPU 41 uses L1, A, L and Equation 2, to calculate an estimated value EIF of visceral fat area. The relationship between the estimated values EIF and the visceral fat area measured in the CT scanning method is as shown in Fig. 10. As shown in this figure, there is a strong correlation between estimated values EIF and the visceral fat areas measured in the CT scanning method. This is because visceral fat area is estimated by calculation using not only the first distance having a strong correlation with visceral fat area but also variable A having a strong correlation with proportion B. Therefore, according to body index apparatus 100, visceral fat area can be estimated with a high degree of accuracy.

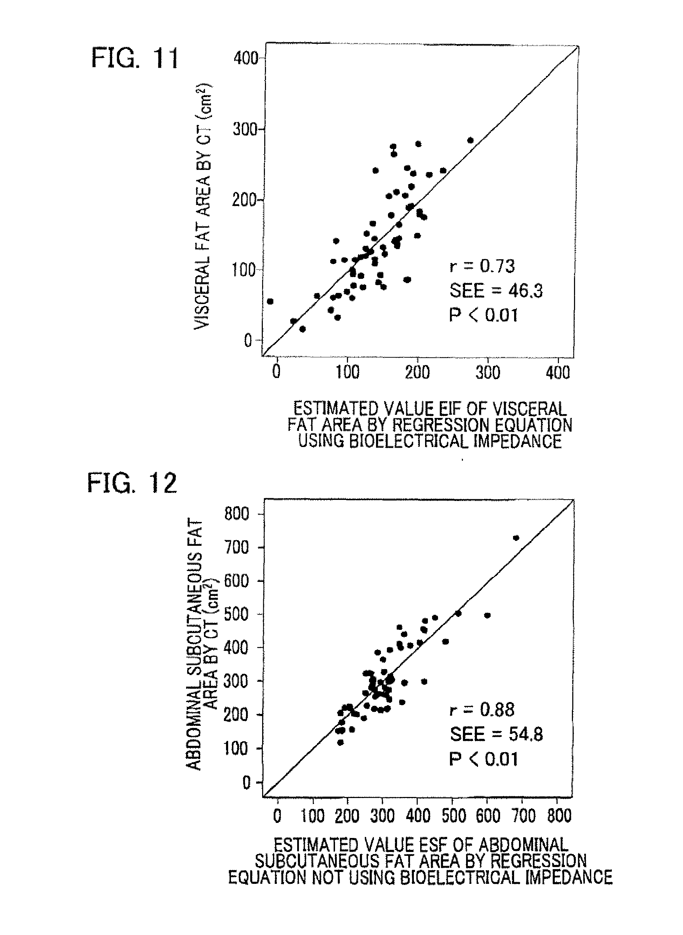

In a case in which bioelectrical impedance data is stored in memory 43, CPU 41 uses L1, A, Z, L, and Equation 3 to calculate an estimated EIF of visceral fat area. The relationship between the estimated values EIF and visceral fat areas measured by the CT scanning method is as shown in Fig. 11. As is clear from the comparison between Figs. 10 and 11, the degree of accuracy in estimation using Equation 3 is improved than the degree of accuracy in estimation using Equation 2.

Estimating abdominal subcutaneous fat area

In estimating abdominal subcutaneous fat area, Equation 4, being a regression equation not using Z, is selected in a case in which bioelectrical impedance data is not stored in memory 43, and Equation 5, being a regression equation, is used in a case in which bioelectrical impedance data is stored in memory 43.

$$ESF = 210.6 - 415.6 * A + 0.363 * (L - L1)^2 \ldots \text{Equation 4}$$

$$ESF = 593.7 - 576.3 * A + 0.003512 * Z * (L - L1)^2 \ldots \text{Equation 5}$$

The coefficients in each term of Equation 4 and Equation 5 are determined so that the degree of accuracy in estimation is the highest when

$$L2 = Ld + Lh \text{ and } L1 = Wmax.$$

In a case in which bioelectrical impedance data is not stored in memory 43, CPU 41 uses L1, A, L, and Equation 4 to calculate an estimated value ESF of abdominal subcutaneous fat area. The relationship between these estimated values ESF and abdominal subcutaneous fat areas measured by the CT scanning method is as shown in Fig. 12. As shown in this figure, there is a high correlation between the estimated values and the abdominal subcutaneous fat areas measured by the CT scanning method. This is because the abdominal subcutaneous fat area is estimated by calculation using not only the first distance having a strong correlation with the abdominal subcutaneous fat area but also variable A having a strong correlation with proportion B. Therefore, according to body index apparatus 100, the abdominal subcutaneous fat area can be estimated with high degree of accuracy.

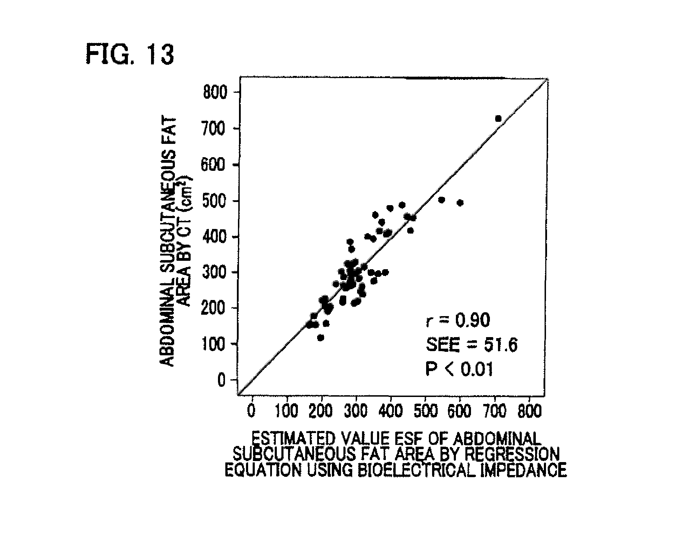

In a case in which bioelectrical impedance data is stored in memory 43, CPU 41 uses L1, A, Z, L, and Equation 5, to calculate an estimated value ESF of abdominal subcutaneous fat area. The relationship between these estimated values ESF and abdominal subcutaneous fat areas measured by the CT scanning method is as shown in Fig. 13. As is clear from the comparison between Figs. 12 and 13, the degree of accuracy in the estimation using Equation 5 is more improved than the degree of accuracy in the estimation using Equation 4.

Estimating obesity type

In the obesity type estimation, Equations 6 and 7 are selected, the Equations 6 and 7 being conditional equations each using variable A.

$$A > 0.95 \ \dots \ \text{Equation 6}$$

$$A \leq 0.9 \ \dots \ \text{Equation 7}$$

The values (thresholds) in the right-hand side of Equations 6 and 7 are determined so that the degree of accuracy in estimation is the highest when $L2 = Ld + Lh$ and $L1 = Wmax$. CPU 41 uses A and Equation 6 to calculate the truth or falsity of Equation 6. In a case in which Equation 6 is true, CPU 41 estimates (determines) that the obesity type of human subject 10 is a visceral fat type. In a case in which Equation 6 is false, CPU 41 uses A and Equation 7 to calculate the truth or falsity of Equation 7. In a case in which Equation 7 is true, CPU 41 estimates (determines) that the obesity type of human subject 10 is a subcutaneous fat type. In a case in which Equations 7 is false, CPU 41 estimates (determines) that the obesity type of human subject 10 is an intermediate type. As described above, because there is a strong correlation between variable A and proportion B, such a method of estimation enables highly accurate estimation of the obesity type.

As described in the foregoing, body index apparatus 100 has a measurer (measurement means) for measuring distance showing the width of abdomen 11 of human subject 10 lying on reference plane S in the supine position at plural measurement points. These measurement points are all fixed, the distances from the measurement points to reference plane S differ from one another, and the measurement points are included in plane G. Furthermore, these measurement points are determined so that the maximum width (the maximum supine width) of widths of abdomen 11 in the above plane is positioned between a measurement point closest to reference plane S and a measurement point that is the most distant from reference plane S. Additionally, body index apparatus 100 has an estimator (estimation means) for estimating an obesity index by calculation using the ratio (variable A) of the second distance (distance indicating the width at the predetermined measurement point) to the first distance indicating the maximum width of distances measured by the measurer at plural measurement points. As described above, because variable A is the feature amount also showing the value of the ratio (proportion B) of abdominal subcutaneous fat to the fat in the abdominal cavity, the feature amount indicating the value of proportion B is measured, thereby enabling the obesity index estimation according to body index apparatus 100.

Furthermore, according to body index apparatus 100, because every measurement using body index apparatus 100 is performed for human subject 10 who is in the supine position, an obesity index can be estimated for a human subject 10 who is bedridden. Moreover, the measurer is a means for measuring distances indicating the abdominal widths at plural measurement points, and the configuration thereof is simple. Therefore, according to body index apparatus 100, the feature amount indicating the value of proportion B can be readily measured. Furthermore, according to body index apparatus 100, because the abdominal circumference, visceral fat area, abdominal subcutaneous fat area, and obesity type can be estimated with a single measurement, obesity can be evaluated from various aspects with a single measurement.

As described previously, subcutaneous fat of abdomen 11 of human subject 10 in the supine position is weighed down to the posterior direction of the abdomen by gravity, it is preferable to measure distance by positioning frame 20 so that plane G is perpendicular to reference plane S that is a plane perpendicular to a vertical direction, from the viewpoint of accuracy of estimation. In this case, it is preferable, additionally, to position frame 20 so that, during the distance measurement, plane G and the inferior-superior direction are at right angles to each other.

Second embodiment

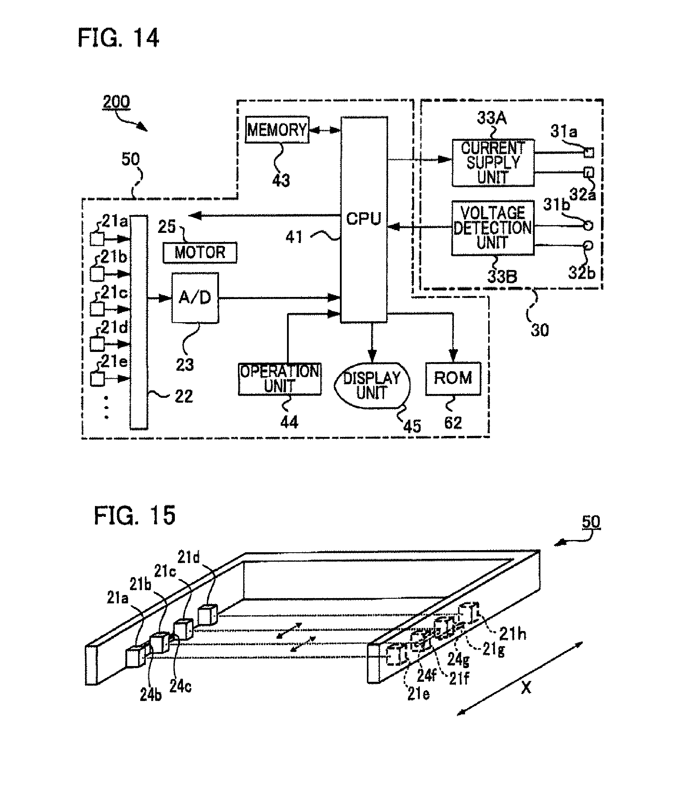

A second embodiment of the present invention is a body index apparatus 200 that a measurer (measurement means) (distance measuring sensor 21, CPU 41, ROM 62, and memory 43) for which two of plural measurement points are movable. In the following, description will be given of body index apparatus 200 with preference to the drawings. Fig. 14 is a block diagram showing an electrical configuration of body index apparatus 200. As shown in this figure, body index apparatus 200 differs from body index apparatus 100 only in that frame 50 is provided in place of frame 20.

Description will be first given as to how frame 50 differs from frame 20.

Fig. 15 is a perspective view showing an external view of frame 50. As shown in this figure, provided on the inner side of frame 50 are a slide rail, or a guide rail, 24b, 24c, 24f, and 24g extending along an X direction. The X direction corresponds to the anteroposterior direction of abdomen 11 during the distance measurement. Distance measuring sensor 21b, 21c, 21f, and 21g each are movable along respective slide rails 24b, 24c, 24f, and 24g. However, in any case, the distance measuring axes of two distance measuring sensors of the same pair of distance measuring sensors correspond with each other.

Additionally, frame 50, as shown in Fig. 14, has a motor 25 for generating power for moving distance measuring sensors 21b, 21c, 21f, and 21g and a slide mechanism (not shown) for using the power generated by motor 25 and for causing distance measuring sensor 21b, 21c, 21f, and 21g to slide along slide rails 24b, 24c, 24f, and 24g for each pair of distance measuring sensors. The slide mechanism is configured so that a distance measuring sensor will not run off from a corresponding slide rail, and so that a distance measuring sensor will not push a next distance measuring sensor.

Furthermore, while frame 20 has ROM 42, frame 50 has a ROM 52. Whereas ROM 42 has stored thereon a first computer program, ROM 62 has stored thereon a computer program (hereinafter referred to as "second computer program") that is different from the first computer program, CPU 41 of body index apparatus 200 executes the second computer program, thereby performing the same operation as CPU 41 of body index apparatus 100, and controls the slide movement of the second-level and third-level pairs of distance measuring sensors in accordance with instructions from an operator.

Specifically, CPU 41 of body index apparatus 200, when a signal instructing the start of a slide movement of the second-level or the third-level pair of distance measuring sensors is supplied from operation unit 44, supplies a signal corresponding to this instruction to motor 25 and the above slide mechanism. Motor 25 and the above slide mechanism are controlled based on this signal. As a result, a pair of distance measuring sensors for which the slide movement is instructed slide to an instructed direction along the two slide rails. Furthermore, CPU 41 of body index apparatus 200, when a signal instructing the end of the slide movement of the pair of distance measuring sensors is supplied from operation unit 44, supplies a signal corresponding to this instruction to motor 25 and to the above slide mechanism. Motor 25 and the above slide mechanism are controlled based on this signal. As a result, all the pairs of distance measuring sensors come to a stop.

As is clear from the foregoing description, according to body index apparatus 200, the second-level and the third-level pairs of distance measuring sensors can be moved in an anteroposterior direction of the abdomen of human subject 10. Therefore, a width shown by the first distance is made closer to the maximum supine width (Wmax). As described above, Equations 1 to 7 are determined so that the degree of accuracy in estimation is the highest when L1 = Wmax. Therefore, according to body index apparatus 200, an obesity index can be estimated with higher degree of accuracy than body index apparatus 100.

Modifications

The present invention can include, in its scope, various embodiments derived by making the following modifications to each of the above embodiments and freely selected combination of these embodiments.

The ratio of the second distance to the first distance, instead of the ratio of the first distance to the second distance, may be used as variable A. Alternatively, the difference between the first distance and the second distance may be used as variable A. Fig. 16 is a scatter diagram showing the relationship between the values of variable A being the difference between the first distance and the second distance, and the values of the ratio (proportion B) of abdominal subcutaneous fat area to fat area in the abdominal cavity, in which the fourth-level pair of distance measuring sensors is a pair of distance measuring sensors at the predetermined measurement point. As is clear from this figure, even in a case in which the difference is used, there is a correlation between variable A and proportion B (the ratio of abdominal subcutaneous fat to fat in the abdominal cavity). Therefore, it is possible to estimate an index relating to obesity by appropriately determining the coefficients in Equations 1 to 5 and the thresholds in Equations 6 and 7. Thus, in the present invention, a freely selected variable that relates to the difference between the first distance and the second distance may be used as variable A. Nevertheless, as is clear from the comparison between Figs. 5 and 16, the correlation between variable A and proportion B is stronger when the ratio is used than when the difference is used. Therefore, using the ratio is preferable in view of improving the degree of accuracy in estimation of an obesity-related index.

A pair of distance measuring sensors other than the fourth-level can be used as a pair of distance measuring sensors at the predetermined measurement point. For example, the first embodiment may be modified so that the third-level pair of distance measuring sensors is used as a pair of distance measuring sensors at the predetermined measurement point. In this case, the second distance (L2) will be a total distance corresponding to the third-level pair of distance measuring sensors. The relationship between the value of variable A being the ratio of the first distance to the second distance and proportion B will be as shown in Fig. 17. As is clear from this figure, there is a correlation between variable A and proportion B even in a case in which the third-level pair of distance measuring sensors is used. Therefore, it is possible to estimate an index relating to obesity by appropriately determining the coefficients in Equations 1 to 5 and the thresholds in Equations 6 and 7. This is the same for a case in which the ratio of the second distance to the first distance is used as variable A and for a case in which the difference between the first distance and the second distance is variable A. However, as is clear from the comparison between Figs. 5 and 17, the correlation between variable A and proportion B is higher when the fourth-level pair of distance measuring sensors is used than when the third-level pair of distance measuring sensors is used. Therefore, in view of improving the degree of accuracy in estimation of an obesity-related index, using the fourth-level distance measuring sensor is preferable.

The number of pairs of distance measuring sensors may be a freely selected plural number. That is, the number of measurement points may be a freely selected plural number. In a case in which the number of measurement points is a freely selected plural number, it is the most appropriate to select a measurement point that is the most distant from reference plane S as the predetermined measurement point for a reason described below.

For example, it is assumed that, as shown in Fig. 3, arranged sequentially from reference plane S are a first measurement point (distance measuring sensors 21a and 21e), a second measurement point (distance measuring sensors 21b and 21f), a third measurement point (distance measuring sensors 21c and 21g), and a fourth measurement point (distance measuring sensors 21d and 21h). It is additionally assumed that a width indicated by the second distance is longer than a width indicated by the first distance.

In this case, in a case in which the predetermined measurement point is the second measurement point, the position of the maximum supine width could be either the anterior side (the side of the third measurement point) of the predetermined measurement point or the posterior side (the side of the first measurement point) of the predetermined position. In a case in which the predetermined measurement point is the third measurement point, the position of the maximum supine width could be either the anterior side (the side of the fourth measurement point) of the predetermined measurement point or the posterior side (the side of the second measurement point) of the predetermined position.

In contrast, in a case in which the predetermined measurement point is the first measurement point that is the closest to reference plane S, the position of the maximum supine width could be the anterior side (the side of the second measurement point) of the predetermined measurement point only. Furthermore, in a case in which the predetermined measurement point is the fourth measurement point that is the most distant from reference plane S, the position of the maximum supine width could be the posterior side (the side of the third measurement point) of the predetermined measurement point only.

Thus, by using a measurement point, from among plural measurement points, that is the closest to reference plane S or a measurement point that is the most distant from reference plane S as the predetermined measurement point, candidates for the relative positional relationship between the predetermined measurement point and the maximum supine width can narrowed down in a case in which a width indicated by the first distance is longer than a width indicated by the second distance. This means that the abdominal shape, to be indicated by variable A, of a human subject in the supine position is more specified. Thus, by determining, as the predetermined measurement point, a measurement point, from among plural measurement points, that is the closest to reference plane S or a measurement point that is the most distant from reference plane S, the possibility is reduced of the abdominal shape indicated by variable A being very different from the actual abdominal shape in a case in which a width indicated by the first distance is longer than a width indicated by the second distance.

On the other hand, because the subcutaneous fat in the abdomen of a human subject in the supine position is weighed down to the posterior side of the abdomen due to gravity, a case in which the first distance is longer than a width at a measurement point that is the most distant from reference plane S is more likely to occur than a case in which the first distance is longer than a width at a measurement point that is the closest to reference plane S. In other words, a case in which the width indicated by the first distance is longer than the width indicated by the second distance (distance at the predetermined position) is more likely to occur when a measurement point that is the most distant from reference plane S is the predetermined measurement point in comparison with a case in which a measurement point that is the closest to reference plane S is the predetermine measurement point.

In the second embodiment, the second-level and the third-level pairs of distance measuring sensors are movable in the anteroposterior direction of abdomen 11. Therefore, when a pair of distance measuring sensors other than the fourth-level pair is made the pair of distance measuring sensors at the predetermine measurement point, a movable pair of distance measuring sensors can be the pair of distance measuring sensors at the predetermined measurement point. Therefore, the present invention includes a mode in which the predetermined measurement point is movable in a direction intersecting reference plane S. In this mode, a set of equations corresponding to Equations 1 to 7 should be prepared respectively for plural points within a movable range of the predetermined measurement point. An obesity-related index is estimated by calculation using a set of equations corresponding to a point that is the closest to the predetermine measurement point. Equations corresponding to Equations 1 to 5 are those in which the coefficients of Equations 1 to 5 are changed depending on a corresponding point, and equations corresponding to Equations 6 and 7 are those in which the thresholds of Equations 6 and 7 are changed depending on a corresponding point.

In the second embodiment, the second-level and the third-level pairs of distance measuring sensors are made movable in the anteroposterior direction of abdomen 11, and the first-level and the fourth-level pairs of distance measuring sensors are fixed, but the present invention is not limited thereto. In other words, the present invention includes a mode in which at least one measurement point of plural measurement points is movable in a direction intersecting reference plane S. In view of bringing the width indicated by the first distance closer to the maximum supine width (Wmax), having a greater number of movable measurement points is referred, whereas, in view of reducing the number of equations to be prepared, having a fewer number of movable measurement points is preferred.

In each of the above embodiments, a total distance is calculated for each of the plural measurement points. These sets of total distances (La + Le, Lb + Lf, Lc + Lg, Ld + Lh) are candidates for the first distance and the second distance. However, the present invention is not limited thereto. For example, L - La - Le (L minus La minus Le), L - Lb - Lf (L minus Lb minus Lf), L - Lc - Lg (L minus Lc minus Lg), L - Ld - Lh (L minus Ld minus Lh) may be candidates. That is, a freely selected distance indicating a width of abdomen 11 of human subject 10 lying on reference plane S in the supine position can be used as a candidate for the first distance and the second distance. In each of the above embodiments, the distance (L) between two distance measuring sensors making up each pair of distance measuring sensors 21 is the same for every pair of distance measuring sensors, but the present invention is not limited thereto.

In a case in which a measurement point, from among plural measurement points, that is the most distant from reference plane S is the predetermined measurement point, there is a possibility that a distance indicating a width of zero is measured at the predetermined measurement point. In a case in which a distance indicating the width of zero is the second distance, the distance between abdomen 11 and the measurement point at which the distance indicating the width of zero is measured must be taken into consideration. Otherwise, the degree of accuracy in estimating an obesity-related index is significantly degraded. To prevent such a situation, an estimator (estimation means) capable of performing a first calculation and a second calculation may be used. The first calculation is a calculation for estimating an obesity-related index in a case in which the predetermined measurement point is set to a measurement point that is the most distant from reference plane S, and the second calculation is a calculation for estimating an obesity-related index in a case in which the predetermined measurement point is set to another measurement point than the measurement point that is the most distant from reference plane S. Furthermore, this estimator, in a case in which the distance measured at a measurement point that is the most distant from reference plane S indicates the width of zero, executes the second calculation, and, in a case in which the distance measured at a measurement point that is the most distant from reference plane S indicates the width other than zero, executes the first calculation. Thus, in this mode, because the distance measured at another measurement point than a measurement point that is the most distant from reference plane S is used as the second distance in a case in which the distance measured at the measurement point that is the most distant from reference plane S indicates the width of zero, a situation can be avoided in which the degree of accuracy in estimating an obesity-related index is significantly degraded.

More than one measurement point of the plural measurement points may be used as the predetermined measurement points. In this case, plural sets of the second distance can be obtained, plural sets of equations are used to perform plural sets of calculations, and an estimation result of an obesity-related index is obtained based on plural sets of calculation results. Furthermore, the number of indices to be estimated may be equal to or greater than one and equal to or less than three. In this case, bioelectrical impedance does not have to be measured if neither visceral fat area nor abdominal subcutaneous fat area is included in an index or indices to be estimated.

The difference between the total distance (La + Le, Lb + Lf, Lc + Lg, Ld + Lh) of each pair of distance measuring sensors and the distance (L) between two distance measuring sensors configuring the same pair may be used as a candidate for the first distance and the second distance. Furthermore, the number of pairs of distance measuring sensor is made to be one, so that this pair of distance measuring sensors is moved in the anteroposterior direction of abdomen 11 so as to scan abdomen 11. Furthermore, a distance measuring sensor that is not an optical type may be used. However, if a contact-type distance measuring sensor is used, abdomen 11 is subject to deformation because the distance measuring sensor touches the surface of abdomen 11 in measuring distance. Therefore, a contactless type distance measuring sensor is preferred to a contact type.

Communication between frame 20 or 50 and bioelectrical impedance measurer 30 may be wired. Furthermore, frame 20 or 50 may have, in addition to bioelectrical impedance measurer 30, a main unit provided with CPU 41, ROM 42 or 62, memory 43, operation unit 44, and display unit 45. This main unit communicates, by wire or wirelessly, with each of frame 20 or 50 and bioelectrical impedance measurer 30. In this case, frame 20 or 50 does not have to have a CPU, a ROM, a memory, an operation unit, or a display unit.

**EP 2 335 577 A1**

**Claims**

1. A body index apparatus (100, 200) comprising:

   measurement means (21, 41, 42, and 43) for measuring, at plural measuring points, a distance indicating an abdominal width of a human subject lying on a reference plane in the supine position, the plural measurement points having different distances to the reference plane and being included in a plane intersecting the reference plane; and
   estimation means (41, 42, and 43) for estimating an index relating to obesity, by calculation using a variable relating to a difference between a first distance and a second distance, the first distance corresponding to the maximum width of plural distances at the plural measurement points measured by the measurement means (21,41,42, and 43) and the second distance corresponding to a distance of the width of a predetermined measurement point of the plural measurement points,
   wherein the plural measurement points are determined in such a way that the maximum value of the abdominal widths in the plane intersecting the reference plane is located between a measurement point closest to the reference plane and a measurement point that is the most distant from the reference plane.

2. A body index apparatus (100, 200) according to Claim 1,
   wherein the estimation means (41, 42, and 43) executes calculation using the first distance and the variable.

3. A body index apparatus (100, 200) according to Claim 2, further comprising bioelectrical impedance measurement means (30) for measuring a bioelectrical impedance of the abdomen by bringing plural electrodes into contact with the abdomen,
   wherein the estimation means (41, 42, and 43) executes calculation using the bioelectrical impedance measured by the bioelectrical impedance measurement means (21, 41, 42, and 43), the first distance, and the variable.

4. A body index apparatus (100, 200) according to one of Claims 1 to 3,
   wherein the variable is the ratio between the first distance and the second distance or the difference between the first distance and the second distance.

5. A body index apparatus (100, 200) according to one of Claims 1 to 4,
   wherein the predetermined measurement point is a measurement point that is the most distant from the reference plane among the plural measurement points.

6. A body index apparatus (100,200) according to Claim 5,
   wherein the estimation means (41, 42, and 43) is capable of executing a first calculation in which the index is estimated when the predetermined measurement point is a measurement point that is the most distant from the reference plane and executing a second calculation in which the index is estimated when the predetermined measurement point is another measurement point, and
   wherein the estimation means (41,42, and 43) executes the second calculation in a case in which the distance of a measurement point that is measured by the measurement means (21, 41, 42, and 43) and is the most distant from the reference plane indicates zero, and executes the first calculation in a case in which the distance of a measurement point that is measured by the measurement means (21,41,42, and 43) and is the most distant from the reference plane does not indicate zero.

7. A body index apparatus (200) according to one of Claims 1 to 6,
   wherein at least one measurement point of the plural measurement points is movable in the direction intersecting the reference plane.

8. A body index apparatus (100, 200) according to one of Claims 1 to 7,
   wherein the measurement means (21 41, 42, and 43) measures the distance of the plural measurement points without touching the abdomen.

FIG. 1

FIG. 2

FIG. 3

FIG. 4

FIG. 5

r = 0.58
SEE= 0.092
P < 0.01

VALUE OF RATIO OF ABDOMINAL SUBCUTANEOUS FAT TO FAT IN THE ABDOMINAL CAVITY (VALUE OF PROPORTION B)

VALUE OF VARIABLE A (FOURTH-LEVEL RATIO)

FIG. 6

FIG. 7

FIG. 8

## FIG. 9

Y-axis: ACTUALLY MEASURED VALUE OF ABDOMINAL CIRCUMFERENCE (cm)

r = 0.93
SEE = 4.71
P < 0.01

X-axis: ESTIMATED VALUE EW OF ABDOMINAL CIRCUMFERENCE BY REGRESSION EQUATION NOT USING VARIABLE A

## FIG. 10

Y-axis: VISCERAL FAT AREA BY CT (cm²)

r = 0.64
SEE = 48.6
P < 0.01

X-axis: ESTIMATED VALUE EIF OF VISCERAL FAT AREA BY REGRESSION EQUATION NOT USING BIOELECTRICAL IMPEDANCE

## FIG. 11

VISCERAL FAT AREA BY CT (cm²)

r = 0.73
SEE = 46.3
P < 0.01

ESTIMATED VALUE EIF OF VISCERAL
FAT AREA BY REGRESSION EQUATION
USING BIOELECTRICAL IMPEDANCE

## FIG. 12

ABDOMINAL SUBCUTANEOUS FAT
AREA BY CT (cm²)

r = 0.88
SEE = 54.8
P < 0.01

ESTIMATED VALUE ESF OF ABDOMINAL
SUBCUTANEOUS FAT AREA BY REGRESSION
EQUATION NOT USING BIOELECTRICAL IMPEDANCE

FIG. 13

ABDOMINAL SUBCUTANEOUS FAT AREA BY CT (cm²)

r = 0.90
SEE = 51.6
P < 0.01

ESTIMATED VALUE ESF OF ABDOMINAL
SUBCUTANEOUS FAT AREA BY REGRESSION
EQUATION USING BIOELECTRICAL IMPEDANCE

## FIG. 14

## FIG. 15

FIG. 16

FIG. 17

Europäisches
Patentamt

European
Patent Office

Office européen
des brevets

## EUROPEAN SEARCH REPORT

**Application Number**

EP 10 19 0529

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 1 882 448 A1 (TANITA SEISAKUSHO KK [JP]) 30 January 2008 (2008-01-30)<br>* abstract; figures 1-3 *<br>* paragraphs [0022], [0023], [0028] - [0037], [0039] - [0042], [0056], [0057] * | 1-8 | INV.<br>A61B5/107<br>A61B5/053 |
| X | EP 1 967 136 A1 (TANITA SEISAKUSHO KK [JP]) 10 September 2008 (2008-09-10)<br>* paragraphs [0020] - [0024], [0 33], [0036], [0038], [0041], [0061], [0062]; figures 1-3 * | 1-8 | |
| X,P | WO 2010/103883 A1 (OMRON HEALTHCARE CO LTD [JP]; MURAKAWA YASUAKI [JP]; HAMAGUCHI TAKEHIR) 16 September 2010 (2010-09-16)<br>* abstract; figure 6 *<br>* description passages in connection to figure 6 * | 1 | |
| X,P | WO 2010/107383 A1 (INNOVATOR SKAANE AB [SE]; FLODMARK CARL-ERIK [SE]) 23 September 2010 (2010-09-23)<br>* abstract; claims 1-17; figures 3,4 *<br>* page 14, lines 15-32 *<br>* page 17, lines 21-23 *<br>* page 20, lines 10-32 * | 1 | TECHNICAL FIELDS SEARCHED (IPC)<br><br>A61B |

The present search report has been drawn up for all claims

| Place of search | Date of completion of the search | Examiner |
|---|---|---|
| Munich | 11 April 2011 | Medeiros Gaspar, Ana |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 10 19 0529

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2011

| Patent document cited in search report | | | Publication date | Patent family member(s) | | | Publication date |
|---|---|---|---|---|---|---|---|
| EP 1882448 | | A1 | 30-01-2008 | JP<br>KR<br>US | 2008049114<br>20080009651<br>2008021349 | A<br>A<br>A1 | 06-03-2008<br>29-01-2008<br>24-01-2008 |
| EP 1967136 | | A1 | 10-09-2008 | CN<br>JP<br>JP<br>US | 101259017<br>4413938<br>2008212447<br>2008221476 | A<br>B2<br>A<br>A1 | 10-09-2008<br>10-02-2010<br>18-09-2008<br>11-09-2008 |
| WO 2010103883 | | A1 | 16-09-2010 | JP | 2010207339 | A | 24-09-2010 |
| WO 2010107383 | | A1 | 23-09-2010 | NONE | | | |

For more details about this annex : see Official Journal of the European Patent Office, No. 12/82

**EP 2 335 577 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009022482 A